# EUROPEAN PATENT SPECIFICATION

(11) **EP 0 135 335 B1**
(45) Date of publication and mention of the grant of the patent: **08.11.1989**
(21) Application number: 84305323.2
(22) Date of filing: 06.08.1984
(51) Int. Cl.: C07D 213/75, A61K 31/44

(54) **N-(2,6-disubstituted aromatic)-N'-pyridinyl ureas, processes for their production and pharmaceutical compositions comprising the same**
N-(2,6-Disubstituierte Aryl)-N'-pyridinylharnstoffe, Verfahren zu ihrer Herstellung und sie enthaltende pharmazeutische Zubereitungen
N-(Aryl-2,6-disubstitué)-N'-pyridinyl-urées, procédés pour leur préparation et compositions pharmaceutiques les contenant

(30) Priority: 22.08.1983 US 525512
(43) Date of publication of application: 27.03.1985
(73) Proprietor: WARNER-LAMBERT COMPANY, Morris Plains New Jersey 07950 (US)
(72) Inventor: Lobbestael, Sandra J., Dexter, MI 48130 (US); Nordin, Ivan C., Holland, MI 49423 (US); Fleming, Robert, Milford, OH 45150 (US)
(74) Representative: Jones, Michael Raymond

(56) References cited:
- EP-A- 0 001 979
- EP-A- 0 022 958
- FR-A- 2 155 856
- PROCEEDINGS OF THE ROYAL SOCIETY, vol. 165, ser. B, 1966, pages 245-265, London, GB; M.I. BRUCE et al.: "Cytokinin activity of some substituted ureas and thioureas"

## Description

Despite optimal use of the several antiepileptic drugs marketed in the United States, many patients with epilepsy fail to experience seizure control and others do so only at the expense of significant toxic side effects. In the early 1970's, no convincing evidence had been published that the primary anti-epileptic drugs marketed in the US at that time controlled the seizures of more than 50% or improved more than 75% of the patients with epilepsy. The availability and use of several additional drugs since that time has brought improved seizure control to many patients. Notwithstanding the beneficial effects of the current drugs, there is still a need for new antiepileptic drugs with more selective anticonvulsant effects and less toxicity. E. A. Swinyard, et al., Epilepsia, 19, 409 (1978).

Various substituted phenyl pyridinyl ureas have been described but none having anticonvulsant activity. For example, M. I. Bruce and J. A. Zwar in Proc. Roy. Soc. (London), Sec. B. 165 (999), 245-65 (1966) disclose many N-mono- and N,N'-disubstituted ureas having cytokinin activity. N-(3,4-dichlorophenyl)-N'-3- and 4-pyridinyl ureas show such activity whereas the corresponding 2,5-dichloro compounds were inactive. In general, the authors concluded that phenyl ring substitution enhanced activity with meta substituents providing highest activity and ortho substituents lowest activity.

German patent publication. 2,928,485 also describes various ureas including N-(3-chloro-4-trifluoromethylphenyl)-N'-3- and 4-pyridinyl ureas as being useful for inhibiting lipid absorption.

French patent publication 2,155,856 teaches various 2-pyridinyl ureas including N-(3,4-dichlorophenyl)-N'-2-pyridinyl urea as having anti-inflammatory and analgesic activity.

The present invention relates to novel 2,6-disubstituted phenyl 3- and 4-pyridinyl ureas having valuable anticonvulsant properties and are thus useful for treating epilepsy.

The present invention relates to a compound of the formula 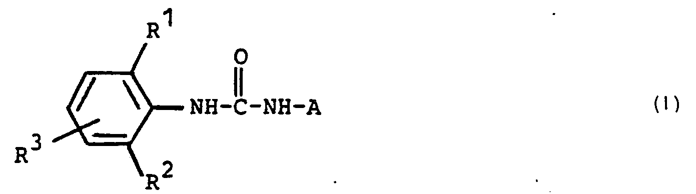 wherein A is 3- or 4-pyridinyl; R¹ and R² are each independently halogen, lower alkyl, lower alkoxy, lower alkanoyl, lower alkoxycarbonyl or nitro, and R³ is hydrogen, halogen, lower alkyl, lower alkoxy, lower alkanoyl, lower alkoxycarbonyl or nitro, or a pharmaceutically acceptable acid addition salt thereof.

The present invention also relates to a process for preparing the compounds of formula I by reacting the appropriate aromatic isocyanate with 3- or 4-aminopyridine.

The present invention further includes a pharmaceutical composition comprising an anticonvulsant effective amount of a compound of formula I together with a pharmaceutically acceptable carrier.

The compounds of the present invention are useful for treating epilepsy in mammals suffering therefrom by administering to such mammals an anticonvulsant effective amount of such a compound.

The term "halogen" as used herein in the definition of the compounds of the formula I includes fluorine, chlorine, bromine, and iodine.

The term "lower" in reference to alkyl, alkoxy, alkanoyl, and alkoxycarbonyl pertains to a straight or branched carbon chain of from 1 to 4 carbon atoms, such as methyl, ethyl, propyl, isopropyl, n-butyl, sec- butyl, iso-butyl, or t-butyl.

The compounds of structural formula I are basic in nature and form pharmaceutically acceptable salts with both organic and inorganic acids. Examples of such acids are acetic, hydrochloric, phosphoric, nitric, sulfuric, fumaric, citric, maleic, malic, and the like. The salts are prepared by contacting the free base form of the pyridinyl urea with an equivalent amount of the desired acid in the conventional manner. The free base forms may be regenerated by treating the salt form with a base. For example, dilute aqueous basic solutions may be utilized. Dilute aqueous sodium hydroxide, sodium carbonate or ammonia are suitable for this purpose. The free base forms differ from their respective salt forms somewhat in certain physical properties such as solubility in polar solvents, but the salts are otherwise equivalent to their respective free base forms for purposes of the invention.

The compounds of the invention of formula I can exist in unsolvated as well as solvated forms, including hydrated forms. In general, the solvated forms, with pharmaceutically acceptable solvents such as water, ethanol, and the like are equivalent to the unsolvated forms for purposes of the invention.

A preferred embodiment of the present invention is a compound of the formula I wherein R³ is hydrogen or a pharmaceutically acceptable acid addition salt thereof.

Another preferred embodiment is a compound of the formula I wherein R¹ and R² are each independently halogen or lower alkyl, or a pharmaceutically acceptable acid addition salt thereof.

Still another preferred embodiment is a compound of the formula I wherein R¹ and R² are each independently chlorine or methyl.

Particular embodiments are the following compounds:
N-(2,6-dichlorophenyl)-N'-4-pyridinyl urea;
N-(2,6-dichlorophenyl)-N'-3-pyridinyl urea;
N-(2,6-dimethylphenyl)-N'-4-pyridinyl urea;
N-(2,6-dimethylphenyl)-N'-3-pyridinyl urea;
N-(2-chloro-6-methylphenyl)-N'-4-pyridinyl urea;
N-(2-chloro-6-methylphenyl)-N'-3-pyridinyl urea;
N-(2,6-diethylphenyl)-N'-4-pyridinyl urea;
N-(2,6-diethylphenyl)-N'-3-pyridinyl urea;
N-(2,6-dimethyl-4-bromophenyl)-N'-4-pyridinyl urea;
N.(2,6-dimethyl-4.bromophenyl).N'-3.pyridinyl urea;
N-(2,4,6-trimethylphenyl)-N'-3-pyridinyl urea;
N-(2,4,6-trimethylphenyl)-N'-4-pyridinyl urea;
N-(2,4,6-trichlorophenyl)-N'-4-pyridinyl urea;
N-(2,4,6-trichlorophenyl)-N'-3-pyridinyl urea, or a pharmaceutically acceptable acid addition salt thereof.

The compounds of formula I may be prepared by reacting an isocyanate of the formula 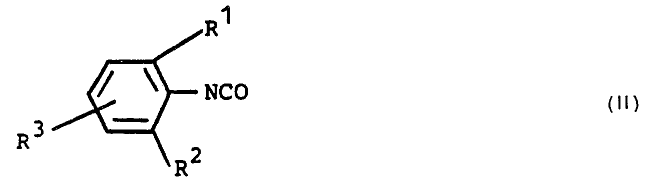 with an equimolar amount of 3- or 4-aminopyridine in an inert solvent, such as tetrahydrofuran, dioxane, and the like, at elevated temperatures, such as at the boiling point of the solvent used.

The starting materials such as the various isocyanates are known and can be purchased commercially or synthesized by known methods.

The compounds of structural formula I are new chemical substances of value as pharmacological agents for the treatment of convulsions in mammals. The term convulsions is intended to mean the characteristic body movements which are associated with the group of chronic central nervous system disorders termed epilepsies. The anticonvulsant activity of representative compounds of formula I was established by the Maximal Electroshock Seizure Test, MES, a standard test procedure described in E. A. Swinyard, et al., Epilepsia, 19, 409 (1978).

An electroshock apparatus was used to deliver shock via ear clips. Trains of square wave D.C. pulses having a pulse duration of 1 mSec and a frequency of 100 pulses per second were delivered at a current strength of 90 mA for 0.2 seconds. The current strength of 90 mA used in this procedure was approximately four times that required to produce seizures in 99% of mice tested and reliably produces seizure in 100% of control mice.

Mice (five in each group) were tested for the presence of neurotoxicity by determining their ability to cling to an inverted wire mesh screen in 60 seconds (NT test).

In the first test of the anticonvulsant screen, groups of five mice each were given intraperitoneal doses of 30,100, and 300 mg/kg of representative compounds of the formula I and the mice tested in the MES and NT tests 0,5, 2, and 4 hours later. The number of mice protected in the MES test are shown in the tables as the numerator (a) and the number of mice that fall off the screen in the NT test are shown in the tables as the denominator (b).

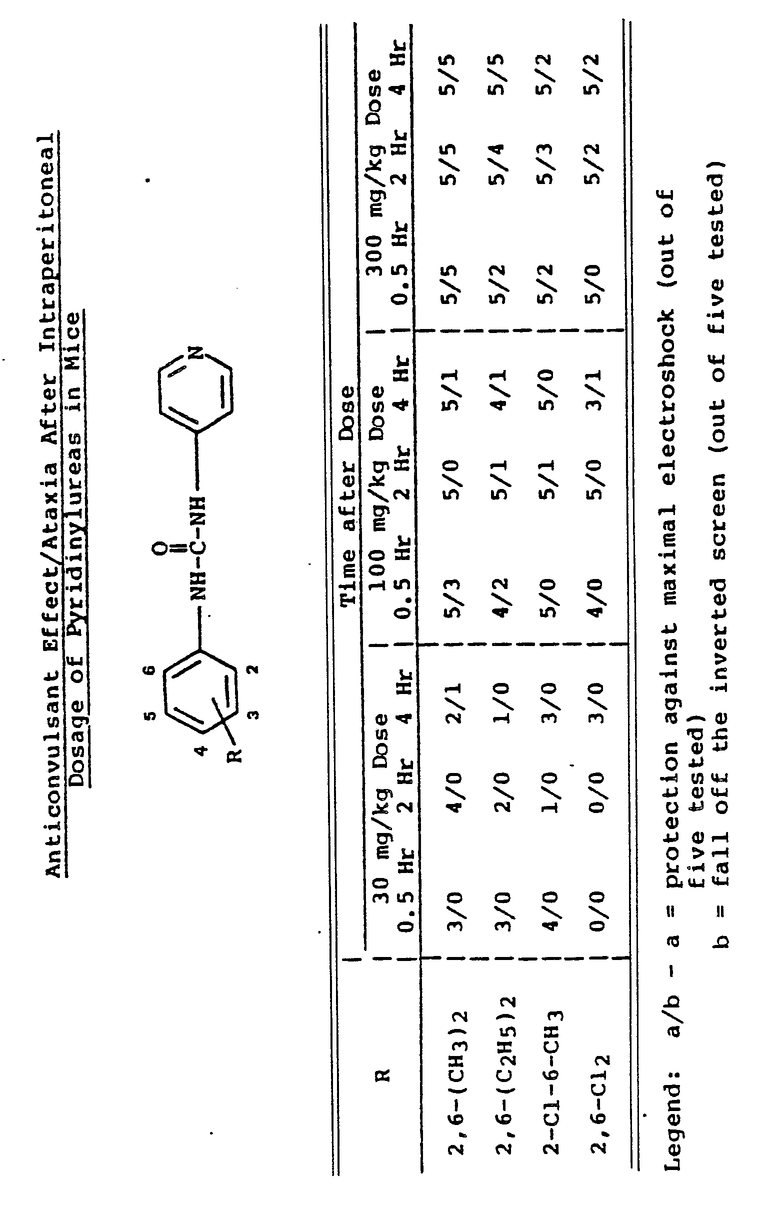

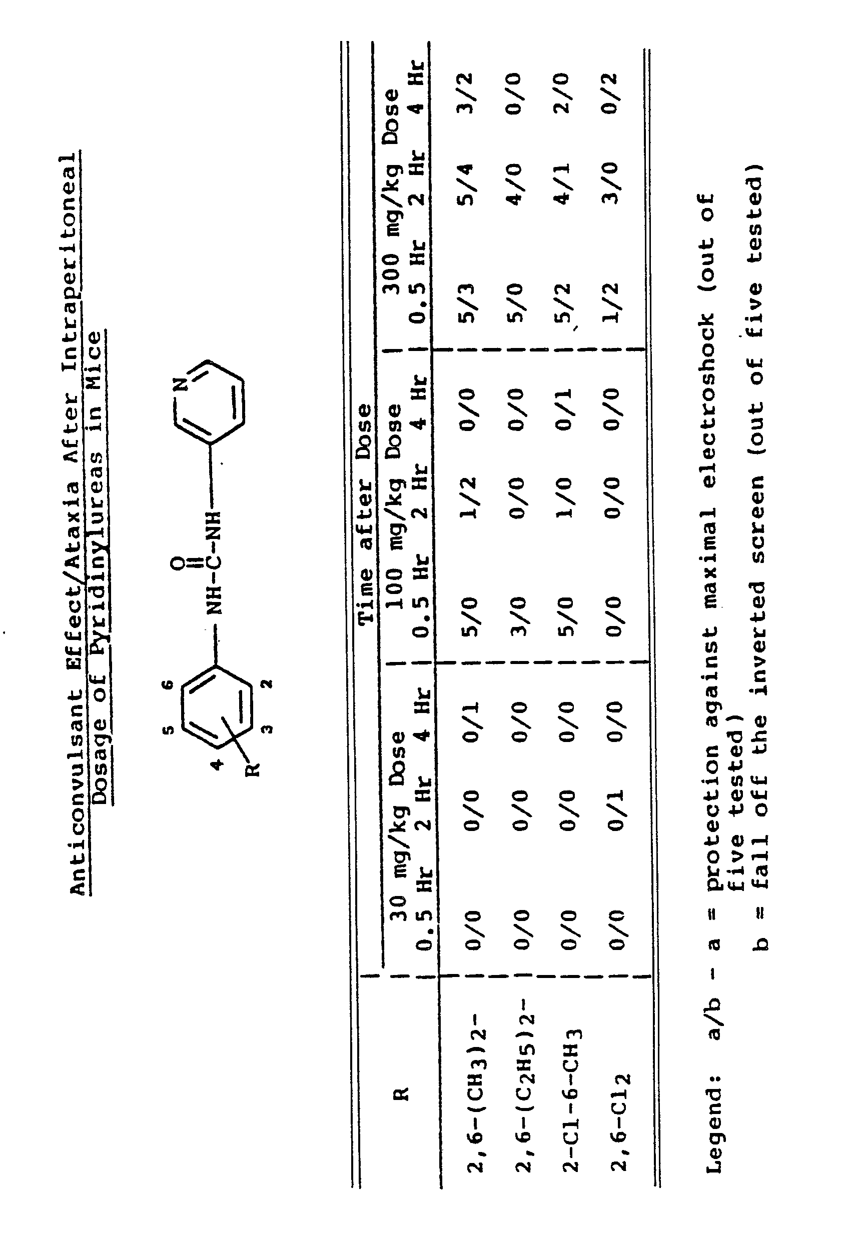

The compounds of structural formula I can be prepared and administered in a wide variety of oral and parenteral dosage forms.

For preparing pharmaceutical compositions from the compounds described by this invention, inert, pharmaceutically acceptable carriers can be either solid or liquid. Solid form preparations include powders, tablets, dispersible granules, capsules, cachets, and suppositories. A solid carrier can be one or more substances which may also act as diluents, flavoring agents, solubilizers, lubricants, suspending agents, binders or tablet disintegrating agents; it can also be an encapsulating material. In powders, the carrier is a finely divided solid which is in admixture with the finely divided active compound of formula I. In the tablet the active compound is mixed with carrier having the necessary binding properties in suitable proportions and compacted in the shape and size desired. The powders and tablets preferably contain from 5 or 10 to about 70 percent of the active ingredient. Suitable solid carriers are magnesium carbonate, magnesium stearate, talc, sugar, lactose, pectin, dextrin, starch, gelatin, tragacanth, methyl cellulose, sodium carboxymethyl cellulose, a low melting wax, cocoa butter, and the like. The term "preparation" is intended to include the formulation of the active compound with encapsulating material as carrier providing a capsule in which the active component (with or without other carriers) is surrounded by carrier, which is thus in association with it. Similarly, cachets are included. Tablets, powders, cachets, and capsules can be used as solid dosage forms suitable for oral administration.

Liquid form preparations include solutions, suspensions, and emulsions. As an example may be mentioned water or water-propylene glycol solutions for parenteral injection. Liquid preparations can also be formulated in solution in aqueous polyethylene glycol solution. Aqueous solutions suitable for oral use can be prepared by dissolving the active component in water and adding suitable colorants, flavors, stabilizing and thickening agents as desired. Aqueous suspensions suitable for oral use can be made by dispersing the finely divided active component in water with viscous material, i.e. natural or synthetic gums, resins, methyl cellulose, sodium carboxymethyl cellulose and other well-known suspending agents. Preferably, the pharmaceutical preparation is in unit dosage form. In such form, the preparation is subdivided into unit doses containing appropriate quantities of the active component. The unit dosage form can be a packaged preparation, the package containing discrete quantities of preparation, for example, packeted tablets, capsules and powders in vials or ampoules. The unit dosage form can also be a capsule, cachet or tablet itself or it can be the appropriate number of any of these in packaged form.

The quantity of active compound in a unit dose of preparation may be varied or adjusted from 1 mg to 100 mg according to the particular application and the potency of the active ingredient.

In therapeutic use as agents for treating convulsions, the compounds utilized in the pharmaceutical method of this invention are administered at the initial dosage of about 0.1 mg to about 21 mg per kilogram daily. A daily dose range of about 0.35 mg to about 12 mg per kilogram is preferred. The dosages, however, may be varied depending upon the requirements of the patient, the severity of the condition being treated and the compound being employed. Determination of the proper dosage for a particular situation is within the skill of the art. Generally, treatment is initiated with smaller dosages which are less than the optimum dose of the compound. Thereafter, the dosage is increased by small increments until the optimum effect under the circumstances is reached. For convenience, the total daily dosage may be divided and administered in portions during the day if desired.

The following nonlimiting examples illustrate the preferred methods for preparing the compounds of the invention.

### Example 1

### N-(2,6-Dichlorophenyl)-N'-4-pyridinyl Urea

A solution of 4.7 g (0.05 mole) of 4-aminopyridine in 300 ml of anhydrous tetrahydrofuran was treated with 9.4 g (0.05 mole) of 2,6-dichlorophenylisocyanate. The solution was heated at reflux for 24 hours, cooled, and concentrated in vacuo to a solid. Recrystallization from aqueous ethanol afforded the crystalline product, mp 217-219°C.

### Example 2

In a similar manner as described in Example 1, the following compounds were prepared by reacting the appropriate isocyanate with 3- or 4-aminopyridine:
N-(2,6-dichlorophenyl)-N'-3-pyridinyl urea, mp 225-227°C;
N-(2,6-dimethylphenyl)-N'-3-pyridinyl urea, mp 190-192°C;
N-(2,6-dimethylphenyl)-N'-4-pyridinyl urea, mp 187-188°C;
N-(2,6-diethylphenyl)-N'-3-pyridinyl urea, mp 196-197°C;
N-(2,6-diethylphenyl)-N'-4-pyridinyl urea, mp 178-180°C;
N-(2-chloro-6-methylphenyl)-N'-3-pyridinyl urea, mp 246-247°C; and
N-(2-chloro-6-methylphenyl)-N'-4-pyridinyl urea, mp 210-212°C.

## Claims (Claims for the following Contracting State(s) : s: BE CH DE FR GB IT LI LU NL SE)

1. A compound having the following general formula: 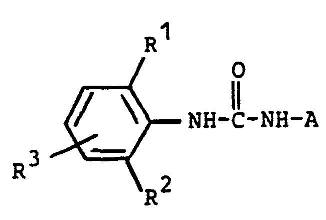 or a pharmaceutically acceptable acid addition salt thereof;
wherein A is a 3- or 4-pyridinyl radical; R¹ and R² are, independently, a halogen atom, a lower alkyl radical, a lower alkoxy radical, a lower alkanoyl radical, a lower alkoxycarbonyl radical or a nitro group; and R³ is a hydrogen atom, a halogen atom, a lower alkyl radical, a lower alkoxy radical, a lower alkanoyl radical, a lower alkoxycarbonyl radical or a nitro group; the term "lower" in reference to alkyl, alkoxy, alkanoyl and alkoxycarbonyl pertaining to the alkyl portion which is straight or branched and of from 1 to 4 carbon atoms.

2. A compound according to Claim 1, wherein R³ is a hydrogen atom.

3. A compound according to Claim 1 or 2, wherein R¹ and R² are, independently, a halogen atom or a lower alkyl radical.

4. A compound according to Claim 3, wherein R¹ and R² are, independently, a chlorine atom or a methyl radical.

5. A compound according to any preceding claim, having the name:
N-(2,6-dichlorophenyl)-N'-4-pyridinyl urea;
N-(2,6-dimethylphenyl)-N'-4-pyridinyl urea;
N-(2-chloro-6-methylphenyl)-N'-4-pyridinyl urea; and
N-(2,6-diethylphenyl)-N'-4-pyridinyl urea;
or a pharmaceutically acceptable acid addition salt thereof.

6. A compound according to any one of Claims 1 to 4, having the name:
N-(2,6-dichlorophenyl)-N'-3-pyridinyl urea;
N-(2,6-dimethylphenyl)-N'-3-pyridinyl urea;
N-(2-chloro-6-methylphenyl)-N'-3-pyridinyl urea; and
N-(2,6-diethylphenyl)-N'-3-pyridinyl urea;
or a pharmaceutically acceptable acid addition salt thereof.

7. A compound according to Claim 1 or to Claim 3 or 4 when appendant to Claim 1, having the name:
N-(2,6-dimethyl-4-bromophenyl)-N'-4-pyridinyl urea;
N-(2,4,6-trimethylphenyl)-N'-4-pyridinyl urea; and
N-(2,4,6-trichlorophenyl)-N'-4-pyridinyl urea;
or a pharmaceutically acceptable acid addition salt thereof.

8. A compound process according to Claim 1 or to Claim 3 or 4 when appendant to Claim 1 having the name:
N-(2,6-dimethyl-4-bromophenyl)-N'-3-pyridinyl urea;
N-(2,4,6-trimethylphenyl)-N'-3-pyridinyl urea; and
N-(2,4,6-trichlorophenyl)-N'-3-pyridinyl urea;
or a pharmaceutically acceptable acid addition salt thereof.

9. A process for preparing a compound as claimed in any preceding claim, which process comprises reacting an isocyanate of the formula: 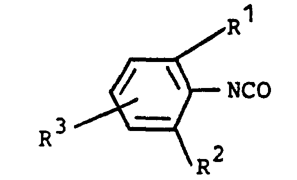 wherein R¹, R² and R³ are as defined in Claim 1, with a 3- or 4-aminopyridine in an inert solvent at elevated temperatures and, if desired, converting, by known methods, the resulting free base to a pharmaceutically acceptable acid addition salt thereof.

10. A pharmaceutical composition which comprises an anti-convulsant effective amount of a compound as claimed in any one of Claims 1 to 8 together with a pharmaceutically acceptable carrier.

## Claims (Claims for the following Contracting State(s) : AT)

1. A process for preparing a compound having the following general formula: 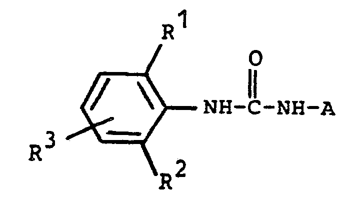 or a pharmaceutically acceptable acid addition salt thereof;
wherein A is a 3- or 4-pyridinyl radical; R¹ and R² are, independently, a halogen atom, a lower alkyl radical, a lower alkoxy radical, a lower alkanoyl radical, a lower alkoxycarbonyl radical or a nitro group; and R³ is a hydrogen atom, a halogen atom, a lower alkyl radical, a lower alkoxy radical, a lower alkanoyl radical, a lower alkoxycarbonyl radical or a nitro group; the term "lower" in reference to alkyl, alkoxy, alkanoyl and alkoxycarbonyl pertaining to the alkyl portion which is straight or branched and of from 1 to 4 carbon atoms;
which process comprises reacting an isocyanate of the formula: 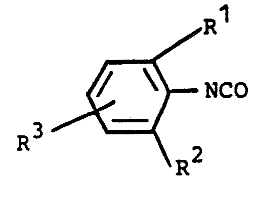 wherein R¹, R² and R³ are as defined above with a 3- or 4-aminopyridine in an inert solvent at elevated temperatures and, if desired, converting, by known methods, the resulting free base to a pharmaceutically acceptable acid addition salt thereof.

2. A process according to Claim 1, wherein R³ is a hydrogen atom.

3. A process according to Claim 1 or 2, wherein R¹ and R² are, independently, a halogen atom or a lower alkyl radical.

4. A process according to Claim 3, wherein R¹ and R² are, independently, a chlorine atom or a methyl radical.

5. A process according to any preceding claim, for preparing a compound having the name:
N-(2,6-dichlorophenyl)-N'-4-pyridinyl urea;
N-(2,6-dimethylphenyl)-N'-4-pyridinyl urea;
N-(2-chloro-6-methylphenyl)-N'-4-pyridinyl urea; and
N-(2,6-diethylphenyl)-N'-4-pyridinyl urea;
or a pharmaceutically acceptable acid addition salt thereof.

6. A process according to any one of Claims 1 to 4, for preparing a compound having the name:
N-(2,6-dichlorophenyl)-N'-3-pyridinyl urea;
N-(2,6-dimethylphenyl)-N'-3-pyridinyl urea;
N-(2-chloro-6-methylphenyl)-N'-3-pyridinyl urea; and
N-(2,6-diethylphenyl)-N'-3-pyridinyl urea;
or a pharmaceutically acceptable acid addition salt thereof.

7. A process according to Claim 1 or to Claim 3 or 4 when appendant to Claim 1, for preparing a compound having the name:
N-(2,6-dimethyl-4-bromophenyl)-N'-4-pyridinyl urea;
N-(2,4,6-trimethylphenyl)-N'-4-pyridinyl urea; and
N-(2,4,6-trichlorophenyl)-N'-4-pyridinyl urea;
or a pharmaceutically acceptable acid addition salt thereof.

8. A process according to Claim 1 or to Claim 3 or 4 when appendant to Claim 1, for preparing a compound having the name:
N-(2,6-dimethyl-4-bromophenyl)-N'-3-pyridinyl urea;
N-(2,4,6-trimethylphenyl)-N'-3-pyridinyl urea; and
N-(2,4,6-trichlorophenyl)-N'-3-pyridinyl urea;
or a pharmaceutically acceptable acid addition salt thereof.

9. A process for preparing a pharmaceutical composition, which process comprises combining an anti- convulsant effective amount of a compound prepared by a process as claimed in any one of Claims 1 to 8 together with a pharmaceutically acceptable carrier.

## Patentansprüche (Patentansprüche für folgende(n) Vertragsstaat(en) : BE CH DE FR GB IT LI LU NL SE)

1. Verbindung mit der folgenden allgemeinen Formel 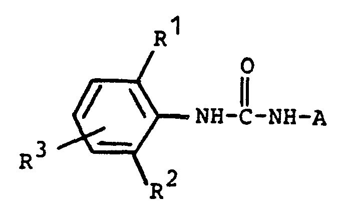 oder ein pharmazeutisch akzeptables Säureadditionssalz davon; worin A ein 3- oder 4-Pyridinylrest ist; R¹ and R² unabhängig voneinander sind: ein Halogenatom, ein Niedrigalkylrest, ein Niedrigalkoxyrest, ein Niedrigalkanoylrest, ein Niedrigalkoxycarbonylrest oder eine Nitrogruppe; und R³ ist: ein Wasserstoffatom, ein Halogenatom, ein Niedrigalkylrest, ein Niedrigalkoxyrest, ein Niedrigalkanoylrest, ein Niedrigalkoxycarbonylrest oder eine Nitrogruppe; der Ausdruck "Niedrig" bezogen auf Alkyl, Alkoxy, Alkanoyl und Alkoxycarbonyl den Alkylanteil, welcher gerad- oder verzweigtkettig ist und von 1 bis 4 Kohlenstoffatome aufweist, betrifft.

2. Verbindung nach Anspruch 1, worin R³ ein Wasserstoffatom ist.

3. Verbindung nach Anspruch 1 oder 2, worin R¹ und R² unabhängig voneinander ein Halogenatom oder ein Niedrigalkylrest sind.

4. Verbindung nach Anspruch 3, worin R' und R² unabhängig voneinander ein Chloratom oder ein Methylrest sind.

5. Verbindung nach irgendeinem vorhergehenden Anspruch mit dem Namen:
N-(2,6-Dichlorphenyl)-N'-4-pyridinylharnstoff;
N-(2,6-Dimethylphenyl)-N'-4-pyridinylharnstoff;
N-(2-Chlor-6-methylphenyl)-N'-pyridinylharnstoff; und
N-(2,6-Diäthylphenyl)-N'-4-pyridinylharnstoff;
oder ein pharmazeutisch akzeptables Säureadditionssalz davon.

6. Verbindung nach irgendeinem der Ansprüche 1 bis 4 mit dem Namen:
N-(2,6-Dichlorphenyl)-N'-3-pyridinylharnstoff;
N-(2,6-Dimethylphenyl)-N'-3-pyridinylharnstoff;
N-(2-Chlor-6-methylphenyl)-N'-3-pyridinylharnstoff; und
N-(2,6-Diäthylphenyl)-N'-3-pyridinylharnstoff;
oder ein pharmazeutisch akzeptables Säureadditionssalz davon.

7. Verbindung nach Anspruch 1 oder Anspruch 3 oder 4, wenn auf Anspruch 1 rückbezogen, mit dem Namen:
N-(2,6-Dimethyl-4-bromphenyl)-N'-4-pyridinylharnstoff;
N-(2,4,6-Trimethylphenyl)-N'-4-pyridinylharnstoff; und
N-(2,4,6-Trichlorphenyl)-N'-4-pyridinylharnstoff;
oder ein pharmazeutisch akzeptables Säureadditionssalz davon.

8. Verbindung nach Anspruch 1 oder Anspruch 3 oder 4, wenn auf Anspruch 1 rückbezogen, mit dem Namen:
N-(2,6-Dimethyl-4-bromphenyl)-N'-3-pyridinylharnstofif;
N-(2,4-6-Trimethylpheny1)-N'-3-pyridinylharnstoff; und
N-(2,4,6-Trichlorphenyl)-N'-3-pyridinylharnstoff;
oder ein pharmazeutisch akzeptables Säureadditionssalz davon.

9. Verfahren zur Herstellung einer wie in irgendeinem vorhergehenden Anspruch beanspruchten Verbindung, welches Verfahren umfaßt: die Umsetzung eines Isocyanats der Formel: 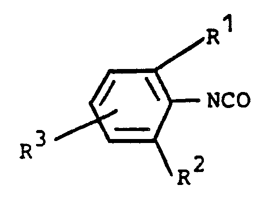 worin R¹, R² und R³ wie im Anspruch 1 definiert sind, mit einem 3- oder 4-Aminopyridin in einem inerten Lösungsmittel, bei erhöhten Temperaturen, und gewünschtenfalls das Überführen der resultierenden freien Base in ein pharmazeutisch akzeptables Säureadditionssalz davon mittels bekannter Methoden.

10. Pharmazeutische Zusammensetzung, welche eine gegen Konvulsionen wirksame Menge einer wie in irgendeinem der Ansprüche 1 bis 8 beanspruchten Verbindung zusammen mit einem pharmazeutisch akzeptablen Träger enthält.

## Patentansprüche (Patentansprüche für folgende(n) Vertragsstaat(en) : AT)

1. Verfahren zur Herstellung einer Verbindung der folgenden allgemeinen Formel: 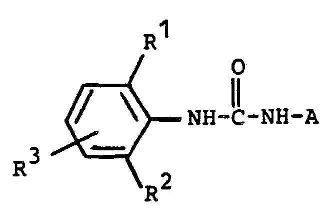 oder eines pharmazeutisch akzeptablen Säureadditionssalzes davon; worin A ein 3- oder 4-Pyridinylrest ist; R¹ and R² unabhängig voneinander sind: ein Halogenatom, ein Niedrigalkylrest, ein Niedrigalkoxyrest, ein Niedrigalkanoyl'rest, ein Niedrigalkoxycarbonylrest oder eine Nitrogruppe; und R³ ist: ein Wasserstoffatom, ein Halogenatom, ein Niedrigalkylrest, ein Niedrigalkoxyrest, ein Niedrigalkanoylrest, ein Niedrigalkoxycarbonylrest oder eine Nitrogruppe; der Ausdruck "Niedrig" bezogen auf Alkyl, Alkoxy, Alkanoyl und Alkoxycarbonyl den Alkylanteil, welcher gerad- oder verzweigtkettig ist und von 1 bis 4 Kohlenstoffatome aufweist, betrifft; welches Verfahren umfaßt: die Umsetzung eines Isocyanats der Formel: 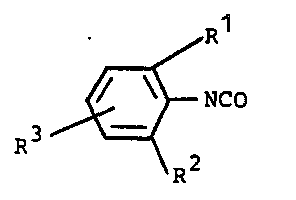 worin R¹, R² und R³ wie oben definiert sind, mit einem 3- oder 4-Aminopyridin in einem inerten Lösungsmittel, bei erhöhten Temperaturen, und gewünschtenfalls das Überführen der resultierenden freien Base in ein pharmazeutisch akzeptables Säureadditionssalz davon mittels bekannter Methoden.

2. Verfahren nach Anspruch 1, worin R³ ein Wasserstoffatom ist.

3. Verfahren nach Anspruch 1 oder 2, worin R¹ und R² unabhängig voneinander ein Halogenatom oder ein Niedrigalkylrest sind.

4. Verfahren nach Anspruch 3, worin R¹ und R² unabhängig voneinander ein Chloratom oder ein Methylrest sind.

5. Verfahren nach irgendeinem vorhergehenden Anspruch zur Herstellung einer Verbindung mit dem Namen:
N-(2,6-Dichlorphenyl)-N'-4-pyridinylharnstoff;
N-(2,6-Dimethylphenyl)-N'-4-pyridinylharnstoff;
N-(2-Chlor-6-methylphenyl)-N'-4-pyridinylharnstoff; und
N-(2,6-Diäthylphenyl)-N'-4-pyridinylharnstoff;
oder eines pharmazeutisch akzeptablen Säureadditionssalzes davon.

6. Verfahren nach irgendeinem der Ansprüche 1 bis 4 zur Herstellung einer Verbindung mit dem Namen:
N-(2,6-Dichlorphenyl)-N'-3-pyridinylharnstoff;
N-(2,6-Dimethylphenyl)-N'-3-pyridinylharnstoff;
N-(2-Chlor-6-methylphenyl)-N'-3-pyridinylharnstoff; und
N-(2,6-Diäthylphenyl)-N'-3-pyridinylharnstoff;
oder eines pharmazeutisch akzeptablen Säureadditionssalzes davon.

7. Verfahren nach Anspruch 1 oder Anspruch 3 oder 4, wenn auf Anspruch 1 rückbezogen, zur Herstellung einer Verbindung mit dem Namen:
N-(2,6-Dimethyl-4-bromphenyl)-N'-4-pyridinylharnstoff;
N-(2,4,6-Trimethylphenyl)-N'-4-pyridinylharnstoff; und
N-(2,4,6-Trich)orpheny))-N'-4-pyridinyiharnstoff;
oder eines pharmazeutisch akzeptablen Säureadditionssalzes davon.

8. Verfahren nach Anspruch 1 oder Anspruch 3 oder 4, wenn auf Anspruch 1 rückbezogen, zur Herstellung einer Verbindung mit dem Namen:
N-(2,6-Dimethyl-4-bromphenyl)-N'-3-pyridinylharnstoff;
N-(2,4,6-Trimethylphenyl)-N'-3-pyridinylharnstoff; und
N-(2,4,6-Trichlorphenyl)-N'-3-pyridinylharnstoff;
oder eines pharmazeutisch akzeptablen Säureadditionssalzes davon.

9. Verfahren zur Herstellung einer pharmazeutischen Zusammensetzung, welches Verfahren die Kombination einer gegen Konvulsionen wirksamen Menge einer durch ein wie in irgendeinem der Ansprüche 1 bis 8 beanspruchtes Verfahren hergestellten Verbindung mit einem pharmazeutisch akzeptablen Träger umfaßt.

## Revendications (Revendications pour l'(les) Etat(s) contractant(s) suivant(s) : BE CH DE FR GB IT LI LU NL SE)

1. Un composé, caractérisé par la formule générale suivante 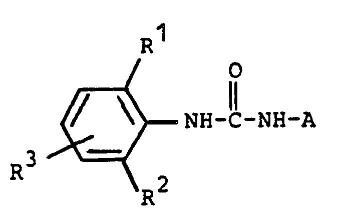 ou un sel de celui-ci acceptable du point de vue pharmaceutique;
dans laquelle A est un groupe 3- ou 4-pyridinyle; R¹ et R² sont indépendamment un atome d'halogène, un groupe alkyle inférieur, alcoxy inférieur, alcanoyle inférieur, alcoxycarbonyle inférieur ou nitro, et R³ est un atome d'hydrogène, d'halogène, un groupe alkyle inférieur, alcoxy inférieur, alcanoyle inférieur, alcoxycarbonyle inférieur ou nitro; le terme "inférieur" en référence aux groupes alkyle, alcoxy, alcanoyle et alcoxycarbonyle concernant la partie alkyle qui est droite ou ramifiée et contient de 1 à 4 atomes de carbone.

2. Un composé suivant la revendication 1, caractérisé en ce que R³ est un atome d'hydrogène.

3. Un composé suivant la revendication 1 ou la revendication 2, caractérisé en ce que R¹ et R² sont indépendamment un atome d'halogène ou un groupe alkyle inférieur.

4. Un composé suivant la revendication 3, caractérisé en ce que R¹ et R² sont indépendamment un atome de chlore ou un groupe méthyle.

5. Un composé suivant l'une quelconque des revendications précédentes, caractérisé en ce qu'il s'agit de:

la N-(2,6-dichlorophényl)-N'-4-pyridinyl urée;
la N-(2,6-diméthylphényl)-N'-4-pyridinyl urée;
la N-(2-chloro-6-méthylphényl)-N'-4-pyridinyl urée;
la N-(2,6-diéthylphényl)-N'-4-pyridinyl urée;
ou d'un de leurs sels d'addition acides acceptables du point de vue pharmaceutique.

6. Un composé suivant l'une quelconque des revendications 1 à 4, caractérisé en ce qu'il s'agit de:
la N-(2,6-dichlorophényl)-N'-3-pyridinyl urée;
la N-(2,6-diméthylphényl)-N'-3-pyridinyl urée;
la N-(2-chloro-6-méthylphényl)-N'-3-pyridinyl urée;
la N-(2,6-diéthylphényl)-N'-3-pyridinyl urée;
ou d'un de leurs sels d'addition acides acceptables du point de vue pharmaceutique.

7. Un composé suivant la revendication 1 ou la revendication 3 ou 4 en référence à la revendication 1, caractérisé en ce qu'il s'agit de:
la N-(2,6-diméthyl-4-bromophényl)-N'-4-pyridinyl urée;
la N-(2,4,6-triméthylphényl)-N'-4-pyridinyl urée;
la N-(2,4,6-trichlorophényl)-N'-4-pyridinyl urée;
ou d'un de leurs sels d'addition acides acceptables du point de vue pharmaceutique.

8. Un composé suivant la revendication 1 ou la revendication 3 ou 4 en référence à la revendication 1, caractérisé en ce qu'il s'agit de:
la N-(2,6-diméthyi-4-bromophényi)-N'-3-pyridiny) urée;
la N-(2,4,6-triméthylphényl)-N'-3-pyridinyl urée;
la N-(2,4,6-trichlorophényl)-N'-3-pyridinyl urée;
ou d'un de leurs sels d'addition acides acceptables du point de vue pharmaceutique.

9. Un procédé pour préparer un composé suivant l'une quelconque des revendications précédentes, caractérisé en ce qu'il comprend la réaction d'un isocyanate de formule: 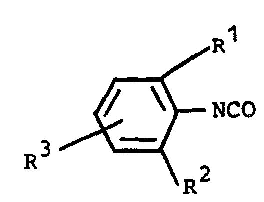 dans laquelle R¹, R² et R³ sont tels que définis dans la revendication 1, avec une 3- ou 4-aminopyridine dans un solvant inerte à des températures élevées, et si cela est désiré, la conversion par des méthodes connues de la base libre résultante en un sel d'addition acide de celle-ci acceptable du point de vue pharmaceutique.

10. Une composition pharmaceutique, caractérisée en ce qu'elle comprend une quantité anticonvulsive d'un composé suivant l'une quelconque des revendications 1 à 8 avec un support acceptable du point de vue pharmaceutique.

## Revendications (Revendications pour l'(les) Etat(s) contractant(s) suivant(s) : AT)

1. Un procédé pour préparer un composé ayant la formule générale suivante 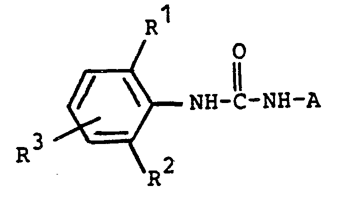 ou un sel de celui-ci acceptable du point de vue pharmaceutique.
dans laquelle A est un groupe 3- ou 4-pyridinyle; R¹ et R² sont indépendamment un atome d'halogène, un groupe alkyle inférieur, alcoxy inférieur, alcanoyle inférieur, alcoxycarbonyle inférieur ou nitro, et R³ est un atome d'hydrogène, d'halogène, un groupe alkyle inférieur, alcoxy inférieur, alcanoyle inférieur, alcoxycarbonyle inférieur ou nitro; le terme "inférieur" en référence aux groupes alkyle, alcoxy, alcanoyle et alcoxycarbonyle concernant la partie alkyle qui est droite ou ramifiée et contient de 1 à 4 atomes de carbone; caractérisé en ce qu'il comprend la réaction d'un isocyanate de formule: 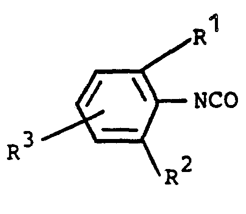 dans laquelle R¹, R² et R³ sont tels que définis plus haut, avec une 3- ou 4-aminopyridine dans un solvant inerte à des températures élevées, et si cela est désiré, la conversion par des méthodes connues de la base libre résultante en un sel d'addition acide de celle-ci acceptable du point de vue pharmaceutique.

2. Un procédé suivant la revendication 1, caractérisé en ce que R³ est un atome d'hydrogène.

3. Un procédé suivant la revendication 1 ou la revendication 2, caractérisé en ce que R¹ et R² sont indépendamment un atome d'halogène ou un groupe alkyle inférieur.

4. Un procédé suivant la revendication 3, caractérisé en ce que R¹ et R² sont indépendamment un atome de chlore ou un groupe méthyle.

5. Un procédé suivant l'une quelconque des revendications précédentes, caractérisé en ce qu'il prépare:
la N-(2,6-dichlorophényl)-N'-4-pyridinyl urée;
la N-(2,6-diméthylphényl)-N'-4-pyridinyl urée;
la N-(2-chloro-6-méthylphényl)-N'-4-pyridinyl urée;
la N-(2,6-diéthylphényl)-N'-4-pyridinyl urée;
ou l'un de leurs sels d'addition acides acceptables du point de vue pharmaceutique.

6. Un procédé suivant l'une quelconque des revendications 1 à 4, caractérisé en ce qu'il prépare:
la N-(2,6-dichlorophényl)-N'-3-pyridinyl urée;
la N-(2,6-diméthylphényl)-N'-3-pyridinyl urée;
la N-(2-chloro-6-méthylphényl)-N'-3-pyridinyl urée;
la N-(2,6-diéthylphényl)-N'-3-pyridinyl urée;
ou l'un de leurs sels d'addition acides acceptables du point de vue pharmaceutique.

7. Un procédé suivant la revendication 1 ou la revendication 3 ou 4 en référence à la revendication 1, caractérisé en ce qu'il prépare:
la N-(2,6-diméthyl-4-bromophényl)-N'-4-pyridinyl urée;
la N-(2,4,6-triméthylphényl)-N'-4-pyridinyl urée;
la N-(2,4,6-trichlorophényl)-N'-4-pyridinyl urée;
ou l'un de leurs sels d'addition acides acceptables du point de vue pharmaceutique.

8. Un procédé suivant la revendication 1 ou la revendication 3 ou 4 en référence à la revendication 1, caractérisé en ce qu'il prépare:
la N-(2,6-diméthyl-4-bromophényl)-N'-3-pyridinyl urée;
la N-(2,4,6-triméthylphényl)-N'-3-pyridinyl urée;
la N-(2,4,6-trichlorophényl)-N'-3-pyridinyl urée;
ou l'un de leurs sels d'addition acides acceptables du point de vue pharmaceutique.

9. Un procédé pour préparer une composition pharmaceutique, caractérisé en ce qu'il comprend la combinaison d'une quantité anticonvulsive d'un composé suivant l'une quelconque des revendications 1 à 8 avec un support acceptable du point de vue pharmaceutique.
